(19) Europäisches Patentamt
European Patent Office
Office européen des brevets

(11) Veröffentlichungsnummer: **0 293 817**
**B1**

(12) # EUROPÄISCHE PATENTSCHRIFT

(45) Veröffentlichungstag der Patentschrift: **01.08.90**

(51) Int. Cl.[5]: **C07C 327/14,** A01N 37/08

(21) Anmeldenummer: **88108664.9**

(22) Anmeldetag: **31.05.88**

(54) **Cyclohexenonverbindungen, ein Verfahren zu ihrer Herstellung sowie ihre Verwendung als Herbizide oder das Pflanzenwachstum regulierende Mittel.**

(30) Priorität: **05.06.87 DE 3718899**

(43) Veröffentlichungstag der Anmeldung: **07.12.88 Patentblatt 88/49**

(45) Bekanntmachung des Hinweises auf die Patenterteilung: **01.08.90 Patentblatt 90/31**

(84) Benannte Vertragsstaaten: **AT BE CH DE ES FR GB GR IT LI NL SE**

(56) Entgegenhaltungen: **EP-A- 0 219 343**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Keil, Michael, Dr., Fontanestrasse 4, D-6713 Freinsheim(DE)**
Erfinder: **Schirmer, Ulrich, Dr., Berghalde 79, D-6900 Heidelberg(DE)**
Erfinder: **Kast, Juergen, Dr., Kastanienstrasse 24, D-6737 Boehl-Iggelheim(DE)**
Erfinder: **Kolassa, Dieter, Dr., Moltkestrasse 8, D-6700 Ludwigshafen(DE)**
Erfinder: **Wuerzer, Bruno, Dr., Ruedigerstrasse 13, D-6701 Otterstadt(DE)**
Erfinder: **Meyer, Norbert, Dr., Dossenheimer Weg 22, D-6802 Ladenburg(DE)**
Erfinder: **Rademacher, Wilhelm, Dr., Austrasse 1, D-6703 Limburgerhof(DE)**
Erfinder: **Jung, Johann, Prof. Dr., Hardenburgstrasse 19, D-6703 Limburgerhof(DE)**
Erfinder: **Carlson, Dale R., Dr., 111 Shady Spring Place, Durham, N.C. 27713(US)**

EP 0 293 817 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die herbizide Wirkung von Cyclohexenonverbindungen, die in der Seitenkette in 2-Stellung eine Oximethergruppe enthalten, ist bekannt (US 4 249 937); CA 1 205 813; Adv. Pest. Science, Part 2, Pergamon Press, Zürich, 1978; E.H. Geissbühler, Proc. 4th Intern. Congress of Pesticide Chemistry (IUPAC), 1978, 235). Es ist weiterhin bekannt, daß bestimmte 2-Acyl-3-hydroxycyclohex-2-en-1-one regulierend in das Pflanzenwachstum eingreifen (US 4 560 403, US 4 584 013, US 4 640 706, EP-A-177 450, EP-A-199 658).

Es wurden nun neue Cyclohexenonverbindungen der Formel I gefunden

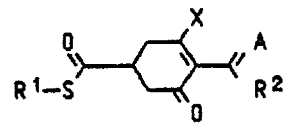

(I)

in der

$R^1$ Wasserstoff; $C_1$–$C_6$-Alkyl unsubstituiert oder substituiert durch $C_1$–$C_4$-Alkoxy, $C_1$–$C_4$-Alkylthio, $C_1$–$C_4$-Dialkylamino, Hydroxy oder Halogen; $C_2$–$C_6$-Alkenyl; $C_3$–$C_6$-Cycloalkyl; Phenyl oder Benzyl, jeweils unsubstituiert oder substituiert durch Halogen, $C_1$–$C_4$-Alkyl, $C_1$–$C_4$-Alkoxy oder Nitro

$R^2$ $C_1$–$C_4$-Alkyl, Cyclopropyl

A Sauerstoff; den Rest $NOR_3$, in dem $R_3$ für $C_1$–$C_4$-Alkyl, $C_3$- oder $C_4$-Alkenyl, $C_3$-oder $C_4$-Alkinyl, $C_2$–$C_4$-Halogenalkyl, $C_3$–$C_4$-Halogenalkenyl, $C_2$–$C_4$-Alkoxyalkyl steht; den Rest $NR^4$, in dem $R^4$ für Wasserstoff, $C_1$–$C_6$-Alkyl, $C_1$–$C_4$-Hydroxyalkyl, $C_1$–$C_4$-Alkoxyalkyl, Benzyl, Phenyl steht

X Hydroxy; Chlor; $C_1$–$C_6$-Alkylthio unsubstituiert oder substituiert durch $C_1$–$C_4$-Alkoxy, $C_1$–$C_4$-Alkylthio, $C_1$–$C_4$-Dialkylamino, Hydroxy oder Halogen; $C_2$–$C_6$-Alkenylthio; $C_3$–$C_6$-Cycloalkylthio; phenylthio oder Benzylthio, jeweils unsubstituiert oder substituiert durch Halogen, $C_1$–$C_4$-Alkoxy oder Nitro

bedeuten. Zur Erfindung gehören auch die entsprechenden, insbesondere landwirtschaftlich brauchbaren Salze der Verbindungen I.

Cyclohexenonverbindungen der Formel I, in der A für den Rest $NOR^3$ steht, besitzen eine vorteilhafte herbizide Wirksamkeit gegen Arten aus der Familie der Gräser (Gramineen).

Cyclohexenonverbindungen der Formel I, in denen A für Sauerstoff oder $NR^4$ steht, zeichnen sich besonders durch vorteilhafte wachstumsregulatorische – z.B. halmverkürzende – Eigenschaften aus.

Zu den Substituenten, die die Verbindungen der Formel I näher beschreiben, ist im einzelnen das folgende zu sagen:

$R^1$ in Formel I bedeutet beispielsweise Wasserstoff, Methyl, Ethyl, n-Hexyl, Isopropyl, tert.-Butyl, Allyl, 2-Methoxyethyl, 2-Ethylthioethyl, 3-Chlorpropyl, 2-Dimethylaminoethyl, 2-Hydroxyethyl, Cyclohexyl, Benzyl, 4-Methyl-benzyl, 4-Chlor-benzyl, Phenyl, p-Tolyl, 4-Chlorphenyl, 4-Methoxyphenyl, 3-Nitrophenyl, bevorzugt hat $R^1$ die Bedeutung Methyl, Ethyl, Phenyl, 4-Chlorphenyl.

$R^2$ in Formel I bedeutet beispielsweise Methyl, Ethyl, n-Propyl, Isopropyl, n-Butyl, Cyclopropyl.

Für den Fall, daß in Formel I A für die Gruppe $NOR^3$ steht, bedeutet $R^3$ beispielsweise Ethyl, Propyl, Allyl, (E)-But-2-en-1-yl, Propargyl, But-2-in-1-yl, (E)-3-Chlorprop-2-en-1-yl, 2-Methoxyethyl.

Für den Fall, daß in Formel I A für die Gruppe $NR^4$ steht, bedeutet $R^4$ beispielsweise Wasserstoff, Methyl, Ethyl, Propyl, Isopropyl, n-Butyl, n-Hexyl, Allyl, 2-Hydroxyethyl, 2-Methoxyethyl, Benzyl, Phenyl.

X in Formel I bedeutet beispielsweise Hydroxy, Chlor, Methylthio, Ethylthio, n-Hexylthio, Isopropylthio, tert.-Butylthio, Allylthio, 2-Methoxyethylthio, 2-Ethylthioethylthiio, 3-Chlorpropylthio, 2-Dimethylamino-ethylthio, 2-Hydroxyethylthio, Cyclohexylthio, Benzylthio, 4-Methylbenzylthio, 4-Chlor-benzylthio, Phenylthio, p-Tolylthio, 4-Chlorphenylthio, 4-Methoxyphenylthio, 3-Nitrophenylthio. Bevorzugt sind Verbindungen, bei denen X die Bedeutung Hydroxy, Chlor, Methylthio, Ethylthio, Phenylthio, 4-Chlorphenylthio hat.

Als Salze der Verbindungen der Formel I kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium, Phosphonium-, Sulfonium, oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die neuen Cyclohexenonverbindungen können z.B. erhalten werden, wenn man eine entsprechende Carbonsäure der Formel II

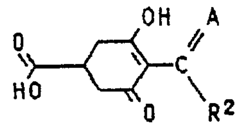

(II)

zunächst mit einem Chlorierungsmittel, gegebenenfalls in Gegenwart einer Base, bei einer Temperatur von −10 bis +120°C, vorzugsweise 20 bis 80°C umsetzt. Bei dieser Umsetzung findet der Austausch der beiden Hydroxylgruppen durch Chlor bevorzugt, d.h. schneller an der seitenständigen (Carboxyl)Gruppierung statt, während die dem Cyclohexenon-Ringsystem zugeordnete Hydroxylgruppe langsamer ausgetauscht wird, unabhängig vom Chlorierungsmittel. Auf diese Weise ist eine größere Mannigfaltigkeit von Verbindungen zugänglich; insbesondere können Cyclohexenon-Verbindungen erhalten werden, die am Ring unverändert Hydroxyl tragen.

Für die Folgeumsetzung, d.h. den Austauxch des Chlors gilt das gleiche: Auch hier findet der Austausch am Säurechlorid schneller statt als am Ring.

Als Chlorierungsmittel verwendet man eines der üblichen, z.B. ein anorganisches oder organisches Säurechlorid, wie Thionylchlorid, Phosgen, Phosphoroxychlorid, Posphorpentachlorid oder Oxalylchlorid.

Als Base kommt eine tertiäre Aminbase, wie Triethylamin, Tributylamin, Tri-2-ethylhexylamin, N,N-Dimethylaminocyclohexyn oder Pyridin in Betracht. Tricarbonylverbindungen und Halogenierungsmittel werden im Molverhältnis 1:1 bis 1:20 eingesetzt, d.h. man verwendet – aus wirtschaftlichen Gründen – das Chlorierungsmittel im Überschuß.

Für den Fall, daß man in Gegenwart einer Base arbeitet, gibt man von dieser einen Überschuß, z.B. 1 bis 20 mol, bezogen auf ein mol der Verbindung zu.

In manchen Fällen kann es von Vorteil sein, in Gegenwart eines inerten Verdünnungsmittels, z.B. Benzol, Toluol, Chloroform, Dichlormethan, 1,2-Dichlorethan, Dioxan, Tetrahydrofuran, N,N-Dimethylformamid oder N-Methylpyrolidon, zu arbeiten.

Die Umsetzung ist nach wenigen Stunden beendet. Durch Einengen erhält man im Regelfall das Säurechlorid III:

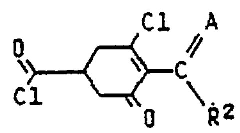

(III)

welches ohne weitere Isolierung mit einem entsprechenden Thiol $R^1$-SH zu einer Verbindung der Formel I umgesetzt werden kann.

Vorteilhaft wird diese Umsetzung in Gegenwart einer tertiären Aminbase, z.B. Triethylamin, N,N-Dimethylaminocyclohexan oder Pyridin durchgeführt.

Das Säurechlorid III und das Thiol $R^1$-SH werden – wiederum aus wirtschaftlichen Erwägungen – im Molverhältnis 1:1 bis 1:20 eingesetzt. Wenn man in Gegenwart einer Base arbeitet, gibt man von dieser ebenfalls einen Überschuß auf das Säurechlorid zu.

Die Substitutionsreaktion kann auch in Gegenwart eines inerten Lösungsmittels, beispielsweise Benzol, Toluol, Chloroform, Dichlormethan, 1,2-Dichlorethan, Dioxan, Tetrahydrofuran, N,N-Dimethylformamid oder N-Methylpyrrolidon vorgenommen werden.

Die Umsetzung ist nach wenigen Stunden beendet; danach kann das Reaktionsprodukt durch Einengen, Aufnehmen in Methylenchlorid und Ausschütteln mit verdünnter Natriumcarbonatlösung und Wasser erhalten werden. Nach Entfernen des Lösungsmittels können die so erhaltenen Rohprodukte säulenchromatographisch über Kieselgel gereinigt werden.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder teirischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentrate, Pasten oder netzbare Pulver (Spritzpulver, Öldispersionen) durch Zusatz von Wasser bereitet werden. Zur Herstellung von Emuldionen, Pasten oder Öldispersionen können die Substanzen als solche oder in einem Öl oder Lösungsittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Edalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalksteink, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,0001 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,001 bis 1, vorzugsweise 0,01 bis 0,1 kg/ha.

Zu den Wirkstoffen können Öle verschiedenen Typs, andere Herbizide bzw. andere Wachstumsregulatoren, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Cyclohexenonderivate der Formel I, bei denen A=Sauerstoff oder $NR^4$ bedeutet, können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und – wegen der relativ geringen Blatt- bzw. Pflanzenmasse – dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren. Dabei können die Cyclohexenonderivatre der Formel I (A=Sauerstoff oder $NR^4$) Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der

Ausbildung von Trenngewebe zwischen Frucht- bzw. Blatt- und Sproßteil der Pflanze ist auch für ein gut kontrollierbares Entblättern von Nutzpflanzen wesentlich.

Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wirkstoffe der Formel I, bei denen A=Sauerstoff oder $NR^4$ bedeutet können den Kulturpflanzen sowohl vom Samen her (als Saatgutbeizmittel) als auch über den Boden, d.h. durch die Wurzel sowie – besonders bevorzugt – durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10g, benötigt.

Für die Blatt- und Bodenbehandlung sind im allgemeinen Gaben von 0,01 bis 5 kg/ha bevorzugt 0,05 bis 2 kg/ha als ausreichend zu betrachten.

Gleichlaufend zur Reduzierung des Längenwachstums stieg auch die Farbintensität der Blätter an. Der erhöhte Chlorophyllgehalt läßt eine ebenfalls erhöhte Photosynteserate und damit eine erhöhte Ertragsbildung erwarten.

Die neuen Mittel der Formel I (A=Sauerstoff oder $NR^4$) können bei diesen Anwendungsformen auch zusammen mit anderen Wirkstoffen verwendet werden, wir z.B. durch Vermischen und Ausbringen mit Herbiziden, Insektiziden, Wachstumsregulatoren und Fungiziden oder auch mit Düngemitteln. Beim Vermischen mit Wachstumsregulatormischungen treten auch synergistische Effekte auf, d.h. die Wirksamkeit des Kombinationsproduktes ist größer als die addierten Wirksamkeiten der Einzelkomponenten.

**Herstellbeispiel**

10.0 g 2-Butyryl-cylcohexan-1,3-dion-5-carbonsäure werden bei 25°C mit 80 ml Oxalylchlorid 16 h gerührt. Die Lösung wird eingeengt und der Rückstand in 120 ml Tetrahydrofuran gelöst. Hierzu wird bei 0°C eine Lösung aus 8,9 g Triethylamin und 5,5 g Ethanthiol in 20 ml Tetrahydrofuran getropft. Nach 3 h Rühren wird eingeengt, mit Dichlormethan/Wasser ausgeschüttelt und eingeengt. Nach Chromatographieren über Kieselgel erhält man 10,1 g 2-Butyryl-3-ethylthio-5-ethylthiocarbonylcyclohex-1-en-3-on als Öl; die Verbindung ist in der nachfolgenden Tabelle als Nr. 46 aufgeführt.

Unter sinngemäßer Abwandlung der vorstehenden Vorschrift, d.h. aus entsprechenden Rohstoffen können beispielsweise folgende, in den Tabellen 1 bis 6 aufgeführte Verbindungen erhalten werden.

Tabelle 1

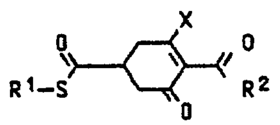

(I)

| Verbindung Nr. | $R^1$ | $R^2$ | X | Schmp. (°C |
|---|---|---|---|---|
| 1 | H | $n-C_3H_7$ | OH | |
| 2 | $CH_3$ | $n-C_3H_7$ | OH | 66-68 |
| 3 | $C_2H_5$ | $n-C_3H_7$ | OH | Harz |
| 4 | $n-C_6H_{13}$ | $n-C_3H_7$ | OH | |
| 5 | H | $C_2H_5$ | OH | |
| 6 | $CH_3$ | $C_2H_5$ | OH | 79-81 |
| 7 | $C_2H_5$ | $C_2H_5$ | OH | 63-65 |
| 8 | $n-C_6H_{13}$ | $C_2H^5$ | OH | |
| 9 | H | Cyclopropyl | OH | |
| 10 | $CH_3$ | Cyclopropyl | OH | |
| 11 | $C_2H_5$ | Cyclopropyl | OH | |
| 12 | $n-C_6H_{13}$ | Cyclopropyl | OH | |
| 13 | $C_2H_5$ | $CH_3$ | OH | 53-55 |
| 14 | H | $n-C_3H_7$ | Cl | |
| 15 | $CH_3$ | $n-C_3H_7$ | Cl | |
| 16 | $C_2H_5$ | $n-C_3H_7$ | Cl | |
| 17 | $n-C_6H_{13}$ | $n-C_3H_7$ | Cl | |
| 18 | H | $C_2H_5$ | Cl | |

Tabelle 1 (Forts.)

| Verbindung Nr. | $R^1$ | $R^2$ | X | Schmp. (°C) |
|---|---|---|---|---|
| 19 | $CH_3$ | $C_2H_5$ | Cl | 82-84 |
| 20 | $C_2H_5$ | $C_2H_5$ | Cl | |
| 21 | n-$C_6H_{13}$ | $C_2H_5$ | Cl | |
| 22 | H | Cyclopropyl | Cl | |
| 23 | $CH_3$ | Cyclopropyl | Cl | |
| 24 | $C_2H_5$ | Cyclopropyl | Cl | |
| 25 | n-$C_6H_{13}$ | Cyclopropyl | Cl | |
| 26 | $C_2H_5$ | $CH_3$ | Cl | |
| 27 | t-$C_4H_9$ | n-$C_3H_7$ | OH | Harz |
| 28 | $CH_3$ | Phenyl | Cl | 98-100 |
| 29 | $CH_3$ | $C_2H_5$ | $NHC_3H_7$ | 68-70 |
| 30 | $CH_2CH_2OCH_3$ | $CH_3$ | OH | Harz |
| 31 | $CH_3$ | $CH_3$ | OH | 69-71 |
| 32 | $CH_2CH_2OCH_3$ | $C_2H_5$ | OH | Harz |
| 33 | $CH_2CH{=}CH_2$ | $C_2H_5$ | OH | Harz |
| 34 | Benzyl | n-$C_3H_7$ | OH | Harz |
| 35 | $C_2H_5$ | $C_4H_9$ | OH | Harz |
| 36 | $CH_2CH_2OCH_3$ | $CH_3$ | OH | Harz |
| 37 | $CH_2CH_2OCH_3$ | $CH_3$ | OH | Harz |
| 38 | t-$C_4H_9$ | $C_2H_5$ | OH | 99-101 |
| 39 | Benzyl | $C_2H_5$ | OH | 56-57 |

Tabelle 2

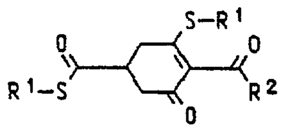

| Verbindung Nr. | $R^1$ | $R^2$ | Schmp ($^0C$) |
|---|---|---|---|
| 40 | $CH_3$ | $CH_3$ | 62-63 |
| 41 | $CH_3$ | $C_2H_5$ | 92-94 |
| 42 | $CH_3$ | $n\text{-}C_3H_7$ | 79-80 |
| 43 | $CH_3$ | $n\text{-}C_4H_9$ | 73-74 |
| 44 | $C_2H_5$ | $CH_3$ | |
| 45 | $C_2H_5$ | $C_2H_5$ | 77-78 |
| 46 | $C_2H_5$ | $n\text{-}C_3H_7$ | Öl |
| 47 | $C_2H_5$ | $n\text{-}C_4H_9$ | |
| 48 | $C_2H_5$ | Cyclopropyl | |
| 49 | $n\text{-}C_6H_{13}$ | $n\text{-}C_3H_7$ | Öl |
| 50 | $CH_2=CH\text{-}CH_2$ | $C_2H_5$ | |
| 51 | $CH_2=CH\text{-}CH_2$ | $n\text{-}C_3H_7$ | Öl |
| 52 | $(CH_3)_2CH$ | $n\text{-}C_3H_7$ | |
| 53 | $(CH_3)_2CH$ | $C_2H_5$ | 39-42 |
| 54 | $(CH_3)_3C$ | $n\text{-}C_3H_7$ | |
| 55 | $CH_3OCH_2CH_2$ | $C_2H_5$ | |
| 56 | $CH_3OCH_2CH_2$ | $n\text{-}C_3O_7$ | Öl |
| 57 | $ClCH_2CH_2CH_2$ | $C_2H_5$ | |
| 58 | $ClCH_2CH_2CH_2$ | $n\text{-}C_3H_7$ | |
| 59 | $(CH_3)_2NCH_2CH_2$ | $C_2H_5$ | |

Tabelle 2 (Forts.)

| Verbindung Nr. | $R^1$ | $R^2$ | Schmp (°C) |
|---|---|---|---|
| 60 | $(CH_3)_2NCH_2CH_2$ | $n-C_3H_7$ | |
| 61 | Cyclohexyl | $n-C_3H_7$ | |
| 62 | Benzyl | $n-C_3H_7$ | |
| 63 | 4-Methylbenzyl | $n-C_3H_7$ | |
| 64 | 4-Chlorbenzyl | $n-C_3H_7$ | |
| 65 | Phenyl | $n-C_3H_7$ | |
| 66 | 4-Methylphenyl | $n-C_3H_7$ | |
| 67 | 4-Chlorphenyl | $n-C_3H_7$ | |
| 68 | 4-Methoxyphenyl | $n-C_3H_7$ | |
| 69 | 3-Nitrophenyl | $n-C_3H_7$ | |
| 70 | Benzyl | $C_2H_5$ | 112-114 |
| 71 | $CH_3$ | $CH_2CH_2CH_2Cl$ | 70-72 |
| 72 | $CH_3$ | Phenyl | 139-140 |
| 73 | $CH_3$ | Cyclopropyl | 103-104 |
| 74 | $CH_3$ | $CH_2OCH_3$ | 79-81 |

Tabelle 3

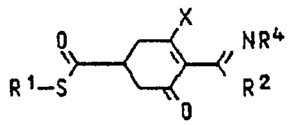

| Verbindung Nr. | $R^1$ | $R^2$ | $R^4$ | X | Schmp (°C) |
|---|---|---|---|---|---|
| 75 | $C_2H_5$ | n-$C_3H_7$ | H | OH | |
| 76 | $C_2H_5$ | n-$C_3H_7$ | n-$C_3H_7$ | OH | 36-38 |
| 77 | $C_2H_5$ | n-$C_3H_7$ | $HOCH_2CH_2$ | OH | |
| 78 | $C_2H_5$ | n-$C_3H_7$ | $CH_3OCH_2CH_2$ | OH | |
| 79 | $C_2H_5$ | n-$C_3H_7$ | $C_6H_5$ | OH | |
| 80 | $C_2H_5$ | n-$C_3H_7$ | $C_6H_5$-$CH_2$ | OH | |
| 81 | $C_2H_5$ | n-$C_3H_7$ | H | Cl | |
| 82 | $C_2H_5$ | n-$C_3H_7$ | n-$C_3H_7$ | Cl | |
| 83 | $C_2H_5$ | n-$C_3H_7$ | $HOCH_2CH_2$ | Cl | |
| 84 | $C_2H_5$ | $CH_3$ | n-$C_3H_7$ | OH | 76-78 |
| 85 | $CH_3$ | $C_2H_5$ | i-$C_3H_7$ | OH | 72-74 |
| 86 | Benzyl | $C_2H_5$ | n-$C_3H_7$ | OH | 42-46 |
| 87 | Ethyl | Cyclopropyl | n-$C_3H_7$ | OH | |

Tabelle 4

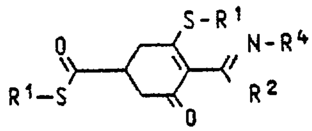

in der

| Verbindung Nr. | $R^1$ | $R^2$ | $R^4$ |
|---|---|---|---|
| 88 | H | $n-C_3H_7$ | $n-C_3H_7$ |
| 89 | $CH_3$ | $n-C_3H_7$ | $n-C_3H_7$ |
| 90 | $C_2H_5$ | $n-C_3H_7$ | H |
| 91 | $C_2H_5$ | $n-C_3H_7$ | $n-C_3H_7$ |
| 92 | $C_2H_5$ | $n-C_3H_7$ | $HO-CH_2CH_2$ |
| 93 | $C_2H_5$ | $n-C_3H_7$ | $CH_3OCH_2CH_2$ |
| 94 | $C_2H_5$ | $n-C_3H_7$ | $C_6H_5$ |
| 95 | $C_2H_5$ | $n-C_3H_7$ | $C_6H_5-CH_2$ |
| 96 | $C_2H_5$ | $n-C_3H_7$ | $CH_2=CH-CH_2$ |
| 97 | $C_2H_5$ | $C_2H_5$ | H |
| 98 | $C_2H_5$ | $C_2H_5$ | $n-C_3H_7$ |
| 99 | $C_2H_5$ | $C_2H_5$ | $HOCH_2CH_2$ |
| 100 | $C_2H_5$ | $C_2H_5$ | $CH_3OCH_2CH_2$ |
| 101 | $C_2H_5$ | $C_2H_5$ | $CH_2=CH-CH_2$ |
| 102 | $n-C_6H_{13}$ | $n-C_3H_7$ | H |
| 103 | $n-C_6H_{13}$ | $n-C_3H_7$ | $n-C_3H_7$ |
| 104 | $CH_2=CH-CH_2$ | $n-C_3H_7$ | H |
| 105 | $CH_2=CH-CH_2$ | $n-C_3H_7$ | $n-C_3H_7$ |
| 106 | $(CH_3)_2CH$ | $n-C_3H_7$ | H |
| 107 | $(CH_3)_2CH$ | $n-C_3H_7$ | $n-C_3H_7$ |
| 108 | $(CH_3)_3C$ | $n-C_3H_7$ | H |
| 109 | $(CH_3)_3C$ | $n-C_3H_7$ | $n-C_3H_7$ |
| 110 | $CH_3OCH_2CH_2$ | $n-C_3H_7$ | H |

Tabelle 5

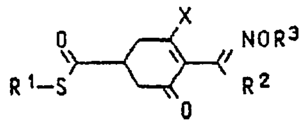

| Verbindung Nr. | $R^1$ | $R^2$ | $R^3$ | X | Schmp. (°C) |
|---|---|---|---|---|---|
| 111 | H | $C_2H_5$ | $C_2H_5$ | OH | |
| 112 | H | $n-C_3H_7$ | $C_2H_5$ | OH | |
| 113 | $CH_3$ | $C_2H_5$ | $C_2H_5$ | OH | 76-78 |
| 114 | $CH_3$ | $n-C_3H_7$ | $C_2H_5$ | OH | |
| 115 | $C_2H_5$ | $C_2H_5$ | $C_2H_5$ | OH | Harz |
| 116 | $C_2H_5$ | $n-C_3H_7$ | $C_2H_5$ | OH | |
| 117 | $C_2H_5$ | Cyclopropyl | $C_2H_5$ | OH | |
| 118 | $C_2H_5$ | $C_2H_5$ | $CH_2=CH-CH_2$ | OH | |
| 119 | $C_2H_5$ | $n-C_3H_7$ | $CH_2=CH-CH_2$ | OH | |
| 120 | $C_2H_5$ | $C_2H_5$ | $E-(ClCH=CH-CH_2)$ | OH | |
| 121 | $C_2H_5$ | $n-C_3H_7$ | $E-(ClCH=CH-CH_2)$ | OH | 39-41 |
| 122 | $C_2H_5$ | $C_2H_5$ | $E-(CH_3CH=CH-CH_2)$ | OH | |
| 123 | $C_2H_5$ | $n-C_3H_7$ | $E-(CH_3CH=CH-CH_2)$ | OH | 32-34 |
| 124 | $n-C_6H_{13}$ | $n-C_3H_7$ | $C_2H_5$ | OH | |
| 125 | $CH_2=CH-CH_2$ | $n-C_3H_7$ | $C_2H_5$ | OH | |
| 126 | $CH_3OCH_2CH_2$ | $n-C_3H_7$ | $C_2H_5$ | OH | Harz |
| 127 | Benzyl | $n-C_3H_7$ | $C_2H_5$ | OH | |
| 128 | 4-Chlorbenzyl | $n-C_3H_7$ | $C_2H_5$ | OH | |
| 129 | Phenyl | $n-C_3H_7$ | $C_2H_5$ | OH | |
| 130 | 4-Chlorphenyl | $n-C_3H_7$ | $C_2H_5$ | OH | |
| 131 | 4-Methoxyphenyl | $n-C_3H_7$ | $C_2H_5$ | OH | |
| 132 | 3-Nitrophenyl | $n-C_3H_7$ | $C_2H_5$ | OH | |
| 133 | $C_2H_5$ | $n-C_3H_7$ | $HC\equiv C$ | OH | |
| 134 | $C_2H_5$ | $n-C_3H_7$ | $CH_3-C\equiv C$ | OH | |
| 135 | $C_2H_5$ | $n-C_3H_7$ | $ClCH_2CH_2$ | OH | |
| 136 | $C_2H_5$ | $n-C_3H_7$ | $CH_3OCH_2CH_2$ | OH | |
| 137 | $C_2H_5$ | $n-C_3H_7$ | $C_2H_5$ | Cl | |
| 138 | $C_2H_5$ | $n-C_3H_7$ | $E-(ClCH=CH-CH_2)$ | Cl | |
| 139 | $C_2H_5$ | $n-C_3H_7$ | $E-(CH_3CH=CH-CH_2)$ | Cl | |
| 140 | $CH_3$ | $CH_3$ | $E-(ClCH=CHCH_2)$ | OH | 67-68 |
| 141 | $CH_3$ | $C_2H_5$ | $C_2H_5$ | OH | 58-60 |
| 142 | Benzyl | $C_2H_5$ | $C_2H_5$ | OH | 39-42 |
| 143 | Benzyl | $C_3H_7$ | $E-(ClCH=CH-CH_2)$ | OH | Harz |
| 144 | Ethyl | Cyclopropyl | $E-(ClCH=CH-CH_2)$ | OH | |

Tabelle 6

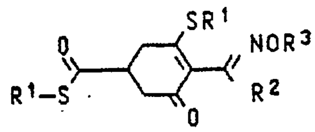

| Verbindung Nr. | $R^1$ | $R^2$ | $R^3$ | Schmp. (°C) |
|---|---|---|---|---|
| 145 | $CH_3$ | $C_2H_5$ | $C_2H_5$ | |
| 146 | $CH_3$ | $n-C_3H_7$ | $C_2H_5$ | |
| 147 | $C_2H_5$ | $C_2H_5$ | $C_2H_5$ | |
| 148 | $C_2H_5$ | $n-C_3H_7$ | $C_2H_5$ | Harz |
| 149 | $C_2H_5$ | $C_2H_5$ | $E-(ClCH=CH-CH_2)$ | |
| 150 | $C_2H_5$ | $n-C_3H_7$ | $E-(ClCH=CH-CH_2)$ | |

Die Cyclohexenonderivate der Formel I können praktisch alle Entwicklungsstadien einer Pflanze verschiedenartig beeinflussen und werden deshalb als Wachstumsregulatoren eingesetzt.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen (ca. 12,5 cm Durchmesser; Volumenca. 500 ml) angezogen.

Bei Bodenbehandlungen wurden die Testsubstanzen in Wäßriger Aufbereitung entweder am Tage der Einsaat (Vorauflaufverfahren) oder nach erfolgter Keimung (Nachauflaufverfahren) auf das Substrat gegossen.

Bei Blattapplikation wurden die zu prüfenden Substanzen in wäßriger Aufbereitung auf die Pflanzen gesprüht (Nachauflauf-Blattbehandlung).

Als Vergleichssubstanz diente der bekannte, handelsübliche Wachstumsregulator Chlormequatchlorid (CCC).

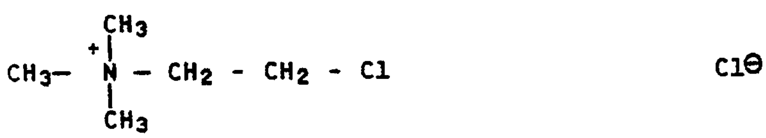

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen 1 bis 4 zu entnehmen:

Tabelle 1

Sommerweizen, Sorte "Kolibri"

Nachauflauf-Blattbehandlung

| Verbindung Nr. | Konz. mg WS/Gef. | Wuchshöhen rel. |
|---|---|---|
| unbehandelt | – | 100 |
| CCC | 1,5 | 79,5 |
| | 6 | 75,1 |
| 33 | 1,5 | 67,8 |
| | 6 | 58,9 |

Tabelle 2

Sommergerste, Sorte "Aramir"

Nachauflauf-Blattbehandlung

| Verbindung Nr. | Konz. mg WS/Gef. | Wuchshöhen rel. |
|---|---|---|
| unbehandelt | – | 100 |
| CCC | 1,5 | 97,6 |
| | 6 | 91,6 |
| 33 | 1,5 | 75,1 |
| | 6 | 61,6 |

Tabelle 3

Reis, "Hihonbare"

Nachauflauf-Blattbehandlung

| Verbindung Nr. | Konz. mg WS/Gef. | Wuchshöhen rel. |
|---|---|---|
| unbehandelt | – | 100 |
| CCC | 1,5 | 100 |
| | 6 | 100 |
| 33 | 1,5 | 84,7 |
| | 6 | 73,7 |

Tabelle 4

Reis, "Hihonbare"

Nachauflauf-Bodenbehandlung

| Verbindung Nr. | Konz. mg WS/Gef. | Wuchshöhen rel. |
|---|---|---|
| unbehandelt | – | 100 |
| CCC | 1,5 | 99,1 |
| | 6 | 97,4 |
| 33 | 1,5 | 95,6 |
| | 6 | 85,0 |

In weiteren Versuchen wurden Gräser bzw. Mischungen von Gräsern in Kunststoffgefäßen (Oberfläche ca 100 $cm^2$) auf einem mit ausreichenden Mengen an Nährstoffen versorgten Kultursubstrat angezogen. Nach einem gleichmäßigen Schnitt des Grases auf ca. 5 cm Länge wurden die zu prüfenden Substanzen in wäßriger Aufbereitung auf die Pflanze gesprüht. Die beobachtete wachstumsregulierende Wirkung wurde bei Versuchsende durch Messung der Sprößlinge belegt. Die so gewonnenen Meßwerte wurden zur Wuchshöhe der unbehandelten Pflanzen in Relation gesetzt.

Als Vergleichssubstanzen dienten:

CCC
Flurprimidol
Mefluidide

Die Einzeldaten sind den Tabellen 5 bis 7 zu entnehmen:

Tabelle 5

Rasen, (Mischung "Berliner Tiergarten").

Nachauflauf-Blattbehandlung (nach Schnitt)

| Verbindung Nr. | Konz. mg WS/Gef. | Wuchshöhen rel. |
|---|---|---|
| unbehandelt | – | 100 |
| CCC | 1,5 | 100 |
| | 6 | 100 |
| 33 | 1,5 | 95,6 |
| | 6 | 85,0 |

Tabelle 6

Bermudagras, (Cynodon dactylon) "Kolibri".

Nachauflauf-Blattbehandlung (nach Schnitt)

Auswertung 2 Wochen nach Behandlung

| Verbindung Nr. | Konz. mg WS/Gef. | Wuchshöhen rel. |
|---|---|---|
| unbehandelt | – | 100 |
| Flurprimidol | 0,2 | 91 |
| | 1,0 | 67 |
| | 5,0 | 67 |
| 33 | 0,2 | 64 |
| | 1,0 | 67 |
| | 5,0 | 56 |

Tabelle 7

Bermudagras, (Cynodon dactylon)

Nachauflauf-Blattbehandlung (nach Schnitt)

Auswertung 6 Wochen nach Behandlung

| Verbindung Nr. | Konz. mg WS/Gef. | Wuchshöhen rel. |
|---|---|---|
| unbehandelt | – | 100 |
| Mefluidide | 0,2 | 89 |
| | 1,0 | 78 |
| | 5,0 | 53* |
| 33 | 0,2 | 82 |
| | 1,0 | 76 |
| | 5,0 | 54 |

* deutliche Blattschäden

## Patentansprüche

1. Cyclohexenonverbindung der Formel I

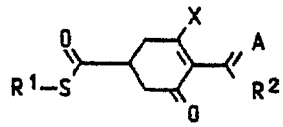

(I)

in der
$R^1$ Wasserstoff; $C_1$–$C_6$-Alkyl unsubstituiert oder substituiert durch $C_1$–$C_4$-Alkoxy, $C_1$–$C_4$-Alkylthio, $C_1$–$C_4$-Dialkylamino, Hydroxy oder Halogen; $C_2$–$C_6$-Alkenyl; $C_3$–$C_6$-Cycloalkyl; Phenyl oder Benzyl unsubstituiert oder substituiert durch Halogen, $C_1$–$C_4$-Alkyl, $C_1$–$C_4$-Alkoxy oder Nitro
$R^2$ $C_1$–$C_4$-Alkyl, Cyclopropyl
A Sauerstoff; den Rest $NOR^3$, in dem $R^3$ für $C_1$–$C_4$-Alkyl, $C_3$- oder $C_4$-Alkenyl, $C_3$- oder $C_4$-Alkinyl, $C_2$–$C_4$-Halogenalkyl, $C_3$–$C_4$-Halogenalkenyl, $C_2$–$C_4$-Alkoxyalkyl steht; den Rest $NR^4$, in dem $R^4$ für Wasserstoff, $C_1$–$C_6$-Alkyl, $C_1$–$C_4$-Hydroxyalkyl, $C_1$–$C_4$-Alkoxyalkyl, Benzyl, Phenyl steht
X Hydroxy; Chlor; $C_1$–$C_6$-Alkylthio unsubstituiert oder substituiert durch $C_1$–$C_4$-Alkoxy, $C_1$–$C_4$-Alkylthio, $C_1$–$C_4$-Dialkylamino, Hydroxy oder Halogen; $C_2$–$C_6$-Alkenylthio; $C_3$–$C_6$-Cycloalkylthio; Phenylthio oder Benzylthio unsubstituiert oder substituiert durch Halogen, $C_1$–$C_4$-Alkoxy oder Nitro
bedeuten, sowie deren landwirtschaftlich brauchbare Salze.

2. Verfahren zur Herstellung von Cyclohexenonverbindungen der Formel I gemäß Anspruch 1, dadurch gekennzeichnet, daß man eine entsprechende Verbindung der Formel II

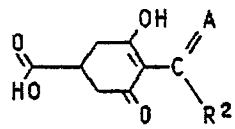

(II)

mit einem Chlorierungsmittel, gegebenenfalls in Gegenwart einer Base, bei einer Temperatur von –10 bis +120°C ein- oder mehrfach umsetzt und die resultierende Chlorverbindung mit einem entsprechenden Thiol der Formel $R^1$–SH, gegebenenfalls in Gegenwart einer Base bei einer Temperatur von –10 bis +120°C umsetzt.

3. Mittel zur Regulierung des Pflanzenwachstums, enthaltend eine Cyclohexenonverbindung der Formel I gemäß Anspruch 1, in der A für Sauerstoff oder einen Rest $NR^4$ steht.

4. Mittel zur Regulierung des Pflanzenwachstums gemäß Anspruch 3, enthaltend inerte Zusatzstoffe.

5. Verfahren zur Regulierung des Pflanzenwachstums, dadurch gekennzeichnet, daß man eine wirksame Menge einer Cyclohexenonverbindung der Formel I gemäß Anspruch 1, in der A für Sauerstoff oder einen Rest $NR^4$ steht, auf Pflanzen und/oder deren Lebensraum einwirken läßt.

## Claims

1. A cyclohexenone compound of the formula I

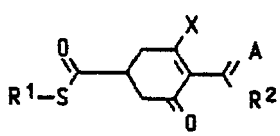

(I)

where $R^1$ is hydrogen; $C_1$–$C_6$-alkyl which is unsubstituted or substituted by $C_1$–$C_4$-alkoxy, $C_1$–$C_4$-alkylthio, $C_1$–$C_4$dialkylamino, hydroxyl or halogen; $C_2$–$C_6$-alkenyl; $C_3$–$C_6$-cycloalkyl;or phenyl or benzyl which is unsubstituted or substituted by halogen, $C_1$–$C_4$-alkyl, $C_1$–$C_4$-alkoxy or nitro, $R^2$ is $C_1$–$C_4$-alkyl or cyclopropyl, A is oxygen; a radical $NOR^3$, where $R^3$ is $C_1$–$C_4$-alkyl, $C_3$ or $C_4$-alkenyl, $C_3$- or $C_4$-alkynyl, $C_2$–$C_4$-haloalkyl, $C_3$- or $C_4$-haloalkenyl or $C_2$–$C_4$-alkoxyalkyl; or a radical $NR_4$, where $R_4$ is hydrogen, $C_1$–$C_6$-alkyl, $C_1$–$C_4$-hydroxyalkyl, $C_1$–$C_4$-alkoxyalkyl, benzyl or phenyl, and X is hydroxyl; chlorine; $C_1$–$C_6$-alkylthio which is unsubstituted or substituted by $C_1$–$C_4$-alkoxy, $C_1$–$C_4$-alkylthio, $C_1$–$C_4$dialkylamino, hydroxyl or halogen; $C_2$–$C_6$-alkenylthio; $C_3$–$C_6$-cycloalkylthio; or phenylthio or benzylthio which is unsubstituted or substituted by halogen, $C_1$-$C_4$-alkoxy or nitro, or an agriculturally usable salt thereof.

2. A process for the preparation of a cyclohexenone compound of the formula I as claimed in claim 1, wherein an appropriate compound of the formula II

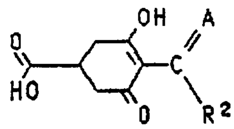

(II)

is reacted once or repeatedly with a chlorinating agent in the presence or absence of a base at from -10 to +120°C, and the resulting chlorine compound is reacted with an appropriate thiol of the formula $R^1$–SH, in the presence or absence of a base at from -10 to +120°C.

3. A plant growth regulator containing a cyclohexenone compound of the formula I as claimed in claim 1, where A is oxygen or a radical $NR^4$.

4. A plant growth regulator as claimed in claim 3, containing inert additives.

5. A method for regulating plant growth, wherein an effective amount of a cyclohexenone compound of the formula I as claimed in claim 1, where A is oxygen or a radical $NR^4$, is allowed to act on plants and/or their habitat.

**Revendications**

1. Dérivés de cyclohéxénone de formule I

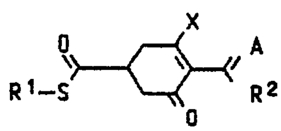

(I)

dans laquelle
$R^1$ représente hydrogène; alkyle en $C_1$–$C_6$ non substitué ou substitué par alcoxy en $C_1$–$C_4$, alkylthio en $C_{1-4}$, dialkylamino en $C_1$–$C_4$, hydroxy ou halogène; alcényle en $C_2$–$C_6$, cycloalkyle en $C_3$–$C_6$: phényle ou benzyle non substitué ou substitué par halogène, alkyle en $C_1$–$C_4$, alcoxy en $C_1$–$C_4$ ou nitro.
$R^2$ représente alkyle en $C_1$–$C_4$, cyclopropyle
A représente oxygène; le reste $NOR^3$, dans lequel $R^3$ est mis pour alkyle en $C_1$–$C_4$ alcényle en $C_3$ ou $C_4$, alcinyle en $C_3$– ou $C_4$, halogénoalkyle en $C_2$–$C_4$, halogénoalcényle en $C_3$–$C_4$, alcoxyalkyle en $C_2$–$C_4$, le reste $NR^4$, dans lequel $R_4$ est mis pour hydrogène, alkyle en $C_1$–$C_6$, hydroxyalkyle en $C_1$–$C_4$, alcoxyalkyle en $C_1$–$C_4$ benzyle, phényle
X représente hydroxy: chloro; alkylthio en $C_1$-$C_6$ non substitué ou substitué par alcoxy en C1–C4, alkylthio en C1–C4, dialkylamino en $C_1$–$C_4$hydroxy ou halogène, alcénylthio en $C_2$–$C_6$; cycloalkylthio en $C_3$–$C_6$: phénylthio ou benzylthio non substitué ou substitué par halogène, alcoxy en $C_1$–$C_4$ ou nitro.
ainsi que leurs sels utilisables en agriculture.

2. Procédé de préparation de dérivés de cyclohéxénone de formule I selon la revendication 1, caractérisé par le fait que l'on fait réagir une ou plusieurs fois, éventuellement en présence d'une base, à une température de –10 à +120°C, avec un agent de chloration un dérivé correspondant de formule II

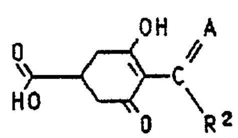

(II)

et on fait le dérivé chloré résultant avec une thiole correspondante de formule $R^1$-SH, éventuellement en présence d'une base à une température de –10 à +120°C.

3. Agent de régulation de la croissance des plantes, contenant un dérivé de cyclohéxénone de formule I selon la revendication 1, dans laquelle A est mis pour oxygène ou un reste $NR^4$.

4. Agent de régulation de la croissance des plantes selon la revendication 3, contenant des additifs inertes.

5. Procédé de régulation de la croissance des plantes, caractérisé par le fait qu'on fait agir sur les plantes et/ou leur biotope une quantité efficace d'un dérivé de cyclohéxénone de formule I selon la revendication 1, dans laquelle A est mis pour oxygène ou un reste $NR^4$.